(19) Europäisches Patentamt
European Patent Office
Office européen des brevets

(11) Publication number: **0 031 407**
**B1**

(12) **EUROPEAN PATENT SPECIFICATION**

(45) Date of publication of patent specification: **22.04.87**

(21) Application number: **80106044.3**

(22) Date of filing: **06.10.80**

(51) Int. Cl.⁴: **C 07 D 219/06,**
C 07 D 219/08,
C 07 D 401/12, C 07 D 413/12
// A61K31/435

(54) **Substituted 3,6 bis-(aminoalkoxy)acridines, and a process for their preparation.**

(30) Priority: **31.10.79 US 89805**

(43) Date of publication of application:
**08.07.81 Bulletin 81/27**

(45) Publication of the grant of the patent:
**22.04.87 Bulletin 87/17**

(84) Designated Contracting States:
**AT BE CH DE FR GB IT LI NL SE**

(56) References cited:
**EP-A-0 012 139**
**DE-A- 490 418**
**US-A-1 727 480**
**US-A-3 740 403**

(73) Proprietor: **AMERICAN CYANAMID COMPANY**
**1937 West Main Street P.O. Box 60**
**Stamford Connecticut 06904-0060 (US)**

(72) Inventor: **Murdock, Keith Chadwick**
**15 Birch Street**
**Pearl River New York 10965 (US)**
Inventor: **Damiani, Martin Robert**
**603 Franklin Turnpike**
**Allendale New Jersey 07401 (US)**
Inventor: **Durr, Frederick Emil**
**387 Jefferson Street**
**Ridgewood New Jersey 07450 (US)**

(74) Representative: **Diehl, Hermann O. Th., Dr.**
**Flüggenstrasse 17**
**D-8000 München 19 (DE)**

**EP 0 031 407 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention is concerned with substituted 3,6-bis-(aminoalkoxy)acridines.

US—A—3740403 concerns 3,6-bis-(dialkylaminoalkoxy)acridines, which show antiviral activity in mice against Columbia SK virus. The known acridines are substituted with a dimethylamino, diethylamino or din-butylamino group. Further prior art is US—A—1,727,480 DE—A—490,418 and EP—A—0012139.

This invention is concerned with new compounds of the formula

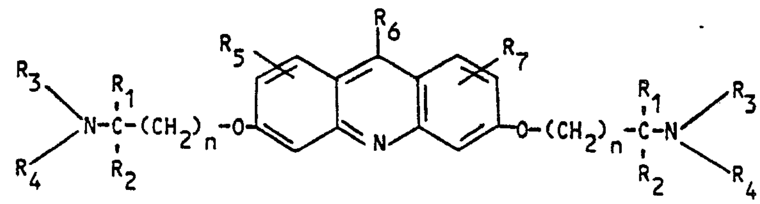

(I)

wherein n is the integer 1, 2 or 3; $R_1$ and $R_2$ are each hydrogen or methyl; $R_3$ and $R_4$ taken together with the associated nitrogen are pyrrolidino, piperidino, morpholino or N-methyl-1-piperazino; and $R_5$, $R_6$ and $R_7$ may be the same or different and are selected from the group consisting of hydrogen, fluoro, chloro, bromo and nitro.

These novel substituted acridines form acid-addition salts with a variety of pharmaceutically acceptable organic and inorganic salt-forming reagents. These acid-addition salts, formed by admixture of the free base form of one of the acridines of the invention with one, two or three equivalents of an acid, suitably in a neutral solvent are formed with such acids as sulfuric, phosphoric, hydrochloric, hydrobromic, nitric, citric, lactic, tartaric, acetic and related acids. For purposes of the present invention, the substituted 3,6-bis(aminoalkoxy)acridine free bases are equivalent to their non-toxic acid-addition salts. The acridines of the invention are substituted with cyclic amine groups. Due to this groups the compounds of the present invention have an antiviral activity, which is different from that of the acridines of the prior art, e.g. that US—A—3740403.

These substituted 3,6-bis(aminoalkoxy)acridines may be readily prepared by treating one mole of the substituted 3,6-acridinediol disodium salt with two moles of an alkyl halide of the formula

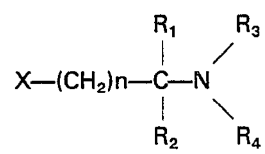

wherein X is chloro or bromo and $R_1$, $R_2$, $R_3$ and $R_4$ are as hereinabove defined. This reaction is preferably carried out in an inert solvent such as dimethylformamide at 75—100°C., for a period of time sufficient for a substantial degree of condensation to occur, particularly for a period of one hour or more. The product may be isolated by removing the inert solvent from the reaction mixture by evaporation, taking up the residue in water and extracting the aqueous phase with diethyl ether. Evaporation of the ether provides the product which may then be purified by advance chromatography.

Alternatively, the compounds may be prepared by reacting 3,6-acridinediol with 1,2- or 1,3-dihaloalkanes or 1-halo-2, or 3-hydroxysulfonyl alkanes in a solvent such as dimethylformamide for 15 minutes to 2 hours. The resulting bis(haloalkoxy)acridine can then be reacted with various amines or ammonia, preferably in a sealed bomb at 80—150°C., for 10—50 hours. The product so isolated is purified as described above.

The 3,6-acridinediol disodium salts may be prepared as described in the literature by treating substituted 3,6-acridinediol with sodium hydride at room temperature in an inert solvent such as dimethylformamide for 15—30 minutes.

The acridine compounds also form complexes with metal atoms such as ferric chloride, zinc chloride, cuprous chloride, potassium hexachloroplatinate, chromic chloride and the like which are considered a part of this invention.

In recent years, the concept of surgical-adjuvant chemotherapy for the treatment of solid neoplasms has become widely accepted by clinical oncologists. Surgery is an effective treatment for the removal of the bulk of a large primary tumor, but often is not applicable to the removal of small, widely scattered metastatic foci of disease. It is recognized that the principal cause of death of cancer patents is the growth of unresolved metastatic tumor foci. Systemic chemotherapy, as an adjunct to surgery, is ideally suited to the elimination of tumor metastases, and has been responsible for much of the progress made in the treatment of human solid tumors.

There are a variety of experimental animal tumor models that are used to assess the therapeutic affects of drugs on primary and metastatic tumor growth. The Lewis lung carcinoma is a highly malignant anaplastic murine carcinoma which when implanted subcutaneously gives rise to a large primary tumor

that metastasizes to the lungs in the early stages of tumor growth. Surgical removal of the primary tumor is not curative unless performed within six days of tumor implantation, thereby preventing metastatic spread of tumor to the lungs. The actual cause of death of the experimental animals, even in the continued presence of the primary tumor, is the growth of the metastatic lung tumors. In this model system, therapeutic drug activity would be indicated by the inhibition of growth of the primary tumor (thereby preventing metastasis formation), or in conjunction with non-curative surgical removal of the primary tumor, by inhibiting the growth of lung metastases. This would be indicated by an increase in median survival time and an increase in the number of long time survivors (i.e. "cures") among drug treated mice when compared with mice that had received non-curative surgery and placebo instead of drug. Alternatively, the Lewis lung carcinoma can be implanted intraveneously in mice to establish "artificial" metastases in the lungs. Therapeutic drug activity, in this instance, would be reflected by an inhibition of the growth of these lung tumor metastases.

The B16 melanoma implated subcutaneously in mice gives rise to a large primary tumor which metastasizes to may distant sites. Surgery is curative when carried out early during the growth of the primary tumor, before metastatic spread of the tumor has occured. Systemic chemotherapy in conjunction with non-curative surgery, has been demonstrated to prevent metastatic tumor spread or to inhibit the growth of metastases formed prior to surgical removal of hte primary tumor. Effective chemotherapy, in the latter instances, is indicated by an increased median survival time or an increased number of long term survivors (i.e. "cures") among drug treated mice when compared with placebo treated mice.

The use of immunomodulants and chemotherapeutic adjuvants constitutes a new therapeutic approach to the treatment of immune deficiencies and cancer and is based on the concept that there are distinctive antigens in or on most tumor cells, such a viral particles and bacteria, that distinguish them from normal host cells, or a particular component of the immune system that can be retulated. A majority of tumor-immunologists favor the view that potentially malignant cells constantly arise but because of their "foreigness" they are normally eliminated by a competent humoral and cellular immune system. Occasionally, however, tumor cells escape this immune surveillance and continue to reproduce and cancer results. The reason for the failure of the normally efficient immune surveillance mechanisms are not fully understood but it is thought that the immune system becomes less effective with increasing age. It is depressed in certain genetic immuno-deficiency diseases, in various bacterial, fungal or viral infections, and in patients undergoing immuno-suppressive therapy. The growth of the neoplasm itself, as well as the various therapeutic modalities designed to treat the disease, e.g., cytotoxic chemotherapy and viraliation, leads to a still greater depression of host resistance and results in an increased susceptibility to both exogenous and indogenous infections and perhaps accounts for the re-initiation of tumor growth, and metastasis which frequently follows treatment induced tumor remission.

If depression of the immune system facilitates the growth of malignancies, regulation of any particular facet of immune responses may help the host to overcome residual cancer cells. Therefore, it is considered desirable to search for chemical agents (i.e., immunoregulants) capable of restoring and stimulating the host's own immune defense mechanisms in order to overcome the deficiencies which account for the increased susceptibility to disease and failure to eradicate the cancer. It is acknowledged that such immunorregulating agents would very likely be incapable of arresting the growth of a large rapidly profilerating tumor but that their clinical utility would derive from their capacity to enhance normal immune surveillance mechanisms in patients whose tumor burden has largely been reduced by conventional surgical, radiotherapeutic or chemotherapeutic methods.

Experimental studies in animals have demonstrated the antitumor potential of a number of immunoregulants including live organisms of bacillus Calmett-Guerin (BCG), heat-killed cells *Corynebacterium parvum*, polynucleotides, and the anthelmintic drug, levamisole. These substances have been shown to stimulate cellular immunity and to produce tumor regressions. Some successes have been claimed in early clinical trails with BCG against malignant melanoma and acute leukemia and with levamisole against lung cancer and beast cancer. Although the antitumor effects produced by these agents have been promising, significant therapeutic benefits have yet to be realized. Since this is a very new therapeutic approach, new drugs and methods of treatment must receive careful clinical evaluation in order to reveal the full potential of these drugs.

Modern research is directed to the discovery of a drug similar to, but more potent than, the known immunoregulants such as levamisole that would be effective in the eradication of tumor cells when used in conjunction with standard therapeutic measures. Stimulators of host resistance may be detected in animal models that can, in fact, detect both immunostimulators and anticancer agents. Mice are put in a condition simulating immunodepression common to cancer patients. This is accomplished by infecting mice either with leukemia virus which produces both leukemia and a disease-related immunodepression or with a transplantalbe mammary tumor. Effective drugs are recognized by their ability to restore or enhance the antibody response in the experimental mice.

The active compounds and novel compositions of the present invention are active as immunomodulators when tested according to the following procedures:

(E) Rauscher leukemia virus

Rauscher leukemia virus is inoculated intraperitoneally into BALB/C mice. The virus inoculum is a

20%(W/V) spleen extract made from 21-day infected spleens of BALB/C mice. All mice are within a three gram weight range, with a minimum weight of 18 g., and all mice are of the same sex, usually male. Sheep red blood cells are injected intraperitoneally on the seventh day. There are 5 mice per test group. The test compound is administered orally on the sixth day as 0.5 ml. (in 0.2% Noble agar in saline) at a dose of 37.5 to 600 mg/kg of body weight, and again on the seventh and eighth day, in the same manner. On the fourteenth day the mice are weighed and bled from the retroorbical sinus. The blood is pooled and the harvested serum is stored at 4°C., for 24 hours. Hemagglutinin tests are performed by standard procedures using the microtiter plate technique. Acceptable hemagglutinin titer for leukemic (immunosuppressed) mice is $\leqq$1:128. Positive control compounds are Poly I:C (polyinosinic acid:-polycytidylic acid) administered intraperitoneally on days +6, +7 and +8. Acceptable positive control hemagglutinin titers are 4-fold higher than the titers obtained in leukemic control mice. The results of this test on typical compounds of this invention appear in Tables I and II.

TABLE I
Rauscher Leukemia Virus
% Reduction in Spleen Size

| DRUG | % Reduction (mg/kg) |
|---|---|
| 3,6-bis[2-(1-piperidinyl)propoxy]acridine trihydrochloride | 74(50) |
| 3,6-bis[2-(1-piperidinyl)ethoxy]acridine trihydrochloride | 84(100) |

TABLE II
Antibody Restoration in Mice With
Rauscher Virus-Induced Leukemia

| DRUG | DOSE mg/kg | ROUTE | SERUM HEMAGGLUTININ TITER/SALINE CONTROL TITER |
|---|---|---|---|
| Poly I:C | 10 | IP | 1024/32 |
| Uninfected Controls | — | | 2048 |
| 3,6-bis[3-(1-piperidinyl)propoxy]acridine ·3HCl | 50 | ORAL | 1024/32 |
| 3,6-bis[2-(1-piperidinyl)ethoxy]acridine ·3HCl | 100 | ORAL | 256/16 |

Protection of mice against lethal virus infection by oral pre-treatment with compounds of the invention

Groups of 20 random-bred Swiss male mice were administered a single oral dose of a compound of the present invention at 400 mg/kg, tilorone hydrochloride at 200 mg/kg, or a saline placebo. At 24, 48, 72 and 96 hours after drug treatment, separate groups of mice were injected s.c. with an $LD_{95}$ dose of the interferon sensitive virus, Columbia SK. Mice were observed for a period of 14 days post-virus infection to determine if drug pre-treatment produced a significant increase in survival relative to the survival of placebo-treated control mice. Typically, protection from lethal infection with Columbia SK virus is associated with induction of interferon. The results appear in Table III.

TABLE III
Antiviral Effect of Drugs Against an Interferon-Sensitive Virus, Columbia SK

| DRUG | DOSE mg/kg (route) | Survivors/Total 14 Days Post Injection |
|---|---|---|
| 3,6-bis[3-(1-piperidinyl)propoxy] acridine ·3HCl | 400 (Oral) | 12/15 |
| 3,6-bis[2-(1-piperidinyl)ethoxy] acridine ·3HCl | 400 (Oral) | 12/15 |
| Poly I:C | | 6/45 |

The novel compounds of this invention do possess antitumor activity in their own right, and are chemotherapeutic adjuvants. In addition, they potentiate the effect of anti-tumor agents and act as adjuvants to surgical therapy.

The compounds of the present invention are effective as immunomodulators (that is, they modulate the immune response) when administered orally in amounts ranging from about 5 mg to about 200 mg per kilogram of body weight per day. A preferred dosage regimen for optimum results would be from about 5 mg to about 50 mg per kilgram of body weight per day, and such dosage units are employed that a total of from about 350 mg to about 3.5 grams of the active compound for a subject of about 70 kg of body weight are administered in a 24-hour period. This dosage regimen may be adjusted to provide the optimum therapeutic response. For example, serveral divided doses may be administered daily or the dose may be proportionally reduced as indicated by the exigencies of the therapeutic situation. A practical advantage of this invention is that the active compound may be administered in any convenient manner such as the oral or buccal routes or it may be incorporated directly in the diet.

The compounds of the present invention may be orally administered, for example, with an inert diluent or with an assimilable edible carrier, or they may be enclosed in hard or soft shell gelatin capsules, or they may be compressed into tablets, or they may be incorporated directly with the food of the diet. For oral therapeutic administration, the active compounds may be incorporated with excipients and used in the form of tablets, troches, capsules, elixirs, suspensions, syrups, wafers, and the like. Such compositions and preparations should contain at least 0.5% of active compound. The percentage of the compositions and preparations may, of course, be varied and may conveniently be between about 2% to about 60% of the weight of the unit. The amount of active ingredient in such therapeutically useful compositions is such that a suitable dosage will be obtained. Preferred compositions or preparations according to the present invention are prepared so that an oral dosage unit form contains between about 50 and 250 milligrams of active compound. The tablets, troches, pills, capsules and the like may also contain the following: a binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier such as a fatty oil. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparbens as preservatives, a dye and flavoring such as cherry or orange flavor. Of course, any material used in preparing any dosage unit form should be pharmaceutically pure and substantially non-toxic in the amounts employed.

The invention will be described in greater detail in conjunction with the following specific examples.

The following examples illustrate the sequence of preparation steps necessary to obtain the compounds of the invention.

Example A

3,6-bis(2-chloroethoxy)acridine hydrochloride

To a mixture of 2.36 g. of a 61.14% dispension of sodium hydride in oil and 5.20 g. of 3,6-acridimediol sulfate (2.1 is added) and 50 ml. of N,N-dimethylformamide. After stirring at room temperature for 40 minutes, 6.35 g. (4.57 ml.) of chloroethyl methanesulfonate are added to the reaction mixture. The mixture is then stirred at room temperature for 2 hours, then at 50°C for 2.5 hours, poured into 250 ml. of ice-water and placed in a freezer overnight. The resulting yellow crystals are collected and dissolved in 100 ml. of chloroform. To this solution is added 6 ml. of 6N hydrochloric acid in isopropanol. Dilution of the chloroform solution with ether gives 2.8 g. of the desired product as yellow crystals, m.p. 228—220°C.

Example B

3,6-bis(2-methylaminoethoxy)acridine trihydrochloride

A suspension of 1.5 g of 3,5-bis(2-chloroethoxy) acridine hydrochloride in 100 ml. of methylamine is heated in a steel bomb at 80°C for 24 hours. The excess methylamine is removed under reduced pressure. The chloroform solution containing the orange residue is washed with two 30 ml. portions of saturated aqueous sodium bicarbonate, then dried over sodium sulfate and filtered. The chloroform is removed, the residue is dissolved in 50 ml. of ethanol and 10 ml. of 6N hydrochloric acid in isopropanol are added. This solution is heated to boiling, water is added until a clear solution is obtained and the solution is cooled, giving 1.25 g. of the desired product as orange crystals, m.p. 267—269°C.

Example C

3,6-bis(2-diethylaminoethoxy)-4,5-dichloroacridine

A 2.148 g. portion of 3,6-bis(2-ethylaminoethoxy)acridine trihydrochloride monohydrate is dissolved in 8 ml. of concentrated sulfuric acid. This solution is cooled in an ice bath and 1.175 g. of N-chlorosuccinimide are added. The mixture is stirred at 0°C for one hour, then at room temperature for 18 hours, poured into 300 ml. of ice water and the pH adjusted to 12 with 10N sodium hydroxide. The mixture is extracted three times with dichloromethane. The extracts are combined, washed with water, dried over anhydrous sodium sulfate, filtered and concentrated in vacuo, giving 1.5 g. of a reddish brown crude product. This product is dissolved in 100 ml. of dichloromethane and filtered through a 5.08 cm. × 6.35 cm. (2 inch × 2.5 inch) bed of alumina. The filter bed is washed with one liter of dichloromethane, then one liter of ethyl acetate. The filtrates are combined, concentrated in vacuo and the residue is crystallized from 10 ml. of hexane giving 1.067 g. of the desired product as yellow crystals, m.p. 83—84°C.

Example D

3,6-bis(3-chloropropoxy)acridine hydrochloride

To a mixture of 11.80 g. of sodium hydride (61.14% oil dispersion) and 26.0 g. of 3,6-acridinediol sulfate (2:1) is added 250 ml. of dry N,N-dimethylformamide. The mixturte is stirred at room temperature for 1.5 hours, then 49.8 g. of 3-chloropropyl-*p*-toluene sulfonate are added. This mixture is stirred at room temperature for 3 hours and 20 minutes, then at 50°C for 2 hours. The volatile materials are removed under reduced pressure at 35—40°C and the residue is quenched with 1200 ml. of ice-cold saturated aqueous sodium bicarbonate solution. The resulting tan crystals are collected and dissolved in 500 ml. of chloroform. A 30 ml. portion of 6N hydrochloric acid in isopropanol is added and the mixture is diluted with ether. The solid is collected and recrystallized from ether, giving the desired product as tan crystals, m.p. 211—213°C.

Example E

3,6-bis(3-diethylaminopropoxy)acridine trihydrochloride

The compound is prepared from 3,6-bis(3-chloropropoxy)acridine hydrochloride and diethylamine as described in Example B except that the mixture is heated at 100°C for 24 hours, giving 1.5 g. of the desired product as yellow crystals, m.p. 235—236°C.

Example F

3,6-bis(2-diethylaminoethoxy)-4,5-dibromoacridine

The procedure of Example C is repeated, using 1.566 g. of N-bromosuccinimide in place of the N-chlorosuccinimide. The crude product is filtered through 6.35 cm. × 3.81 cm. (2.5 inch × 1.5 inch) Magnesol plug using 2 liters of ethyl acetate. The filtrate is concentrated in vacuo and the residue crystallized from 10 ml. of heptane, giving 0.921 g. of the desired product as yellow crystals, m.p. 66—68°C.

The following examples illustrated the invention.

Example 1

3,6-bis[3-(1-piperidinyl)propoxy]acridine

The compound is prepared from 3,6-bis(3-chloropropoxyl)acridine hydrochloride and piperidine as described in Example E, except that the free base is solated.

Example 2

3,6-[3-(1-piperidinyl)propoxyl]acridine trihydrochloride

The trihydrochloride salt of 3,6-bis[3-(1-piperidinyl)-propoxy]acridine is prepared as described in Example B.

Example 3

3,6-[3-(1-pyrrolidinyl)propoxy]acridine

The compound is prepared from 3,6-bis(3-chloropropoxy)acridine hydrochloride and pyrrolidine as described in Example 1.

Example 4

3,6-[3-(4-methyl-1-piperazinyl)propoxy]acridine

The compound is prepared from 3,6-bis(3-chloropropoxy)acridine hydrochloride and N-methylpiperazine as described in Example 1.

Example 5

4,5-dibromo-3,6-bis[2-(1-piperidinyl)ethoxy]acridine

The compound is prepared from 3,6-bis[2-(1-piperidinyl)ethoxy]acridine and N-bromosuccinimide as described in Example F.

Example 6

4,5-dichloro-3,6-[3-(1-piperidinyl)ethoxy]acridine

The compound is prepared from 3,6-bis[2-(1-piperidinyl)ethoxy]acridine and N-chlorosuccinimide as described in Example C.

Example 7

3,6-bis[4-(1-piperidinyl)butoxy]acridine trihydrochloride

The compound is prepared from 3,6-bis(4-chlorobutoxy)acridine hydrochloride and piperidine as described in Example D.

Example 8

3,6-bis[4-(1-piperidinyl)2-methylbutoxy]acridine trihydrochloride

The compound is prepared from 3,6-bis(4-chloro-2-methylbutoxy)acridine hydrochloride and piperidine as described in Example D.

Example 9

3,6-bis[2-(1-pyrrolidinyl)ethoxy]acridine trihydrochloride

The compound is prepared from 3,6-bis(2-chloroethoxy)acridine hydrochloride and pyrrolidine as described in Example D.

Example 10

4,5-dichloro-3,6-bis[2-(1-pyrrolidinyl)ethoxy]acridine

The compound is prepared from 3,6-bis[2-(1-pyrrolidinyl)ethoxy]acridine trihydrochloride and N-chlorosuccinimide as described in Example C.

**Claims for the Contracting States: BE CH DE FR GB IT LI NL SE**

1. A compound selected from the group consisting of those of the formula

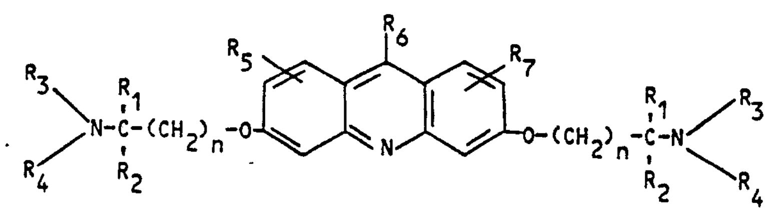

(I)

wherein n is the integer 1, 2 or 3; $R_1$ and $R_2$ are each hydrogen or methyl; $R_3$ and $R_4$ taken together with the associated nitrogen are 1-pyrrolidino, 1-piperidino, 1-morpholino or 4-methyl-1-piperazino; and $R_5$, $R_6$ and $R_7$ may be the same or different and are selected from the group consisting of hydrogen, fluoro, chloro, bromo and nitro; the pharmacologically acceptable metal complexes thereof and the pharmacologically acceptable acid-addition salts thereof.

2. The compounds according to Claim 1; 3,6-bis[2-(1-piperidinyl)ethoxy]acridine and the pharmacologically acceptable metal complexes and acid-addition salts thereof.

3. The compounds according to Claim 1; 3,6-bis[3-(1-piperidinyl)propoxy]acridine and the pharmacologically acceptable metal complexes and acid-addition salts thereof.

4. The compounds according to Claim 1; 3,6-bis[3-(1-pyrrolidinyl)propoxy]acridine and the pharmacologically acceptable metal complexes and acid-addition salts thereof.

5. The compounds according to Claim 1; 3,6-bis[3-(4-methyl-1-piperazinyl)propoxy]acridine and the pharmacologically acceptable metal complexes and acid-addition salts thereof.

6. The compounds according to Claim 1; 4,5-dibromo-3,6-bis[2-(1-piperidinyl)ethoxy]acridine and the pharmacologically acceptable metal complexes and acid-addition salts thereof.

7. The compounds according to Claim 1; 4,5-dichloro-3,6-bis[2-(1-piperidinyl)ethoxy]acridine and the pharmacologically acceptable metal complexes and acid-addition salts thereof.

8. The compounds according to Claim 1; 3,6-bis[4-(1-piperidinyl)butoxy]acridine and the pharmacologically acceptable metal complexes and acid-addition salts thereof.

9. The compounds according to Claim 1; 3,6-bis[2-(1-pyrrolidinyl)ethoxy]acridine and the pharmacologically acceptable metal complexes and acid-addition salts thereof.

10. The compounds according to Claim 1; 4,5-dichloro-3,6-bis[2-(1-pyrrolidinyl)ethoxy]acridine and the pharmacologically acceptable metal complexes and acid-addition salts thereof.

11. The process of preparing compounds of the formula (I) according to Claim 1

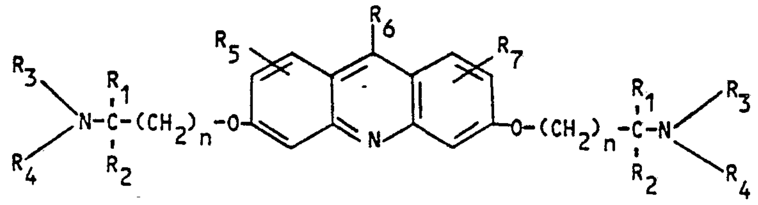

(I)

wherein $R_1$, $R_2$, $R_3$, $R_4$, $R_5$, $R_6$, $R_7$ and n are as defined in claim 1, characterized by reacting a substituted 3,6-acridinediol disodium salt of the formula

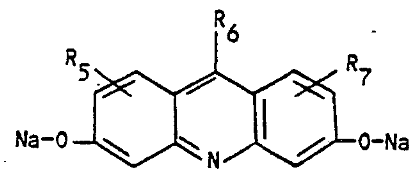

wherein $R_5$, $R_6$ and $R_7$ are as hereinbefore defined with two mole equivalents of an alkyl halide of the formula

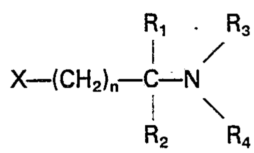

wherein X is chloro or bromo and N, $R_1$, $R_2$ and

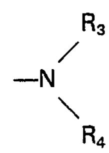

are as hereinbefore defined, in an inert solvent at 75°—100°C for a period of time sufficient for a substantial degree of condensation to occur; and, if desired, forming the metal complexes or acid-addition salts thereof.

12. A pharmaceutical composition comprising a compound according to any one of Claims 1—10 and a pharmacologically acceptable carrier or diluent.

**Claims for the Contracting State: AT**

1. A process for preparing a pharmaceutical composition useful for inducing the regression of tumors in warm-blooded animals and for enhancing the immune system, characterized in that a pharmacologically acceptable carrier or diluent is admixed with compounds of the formula

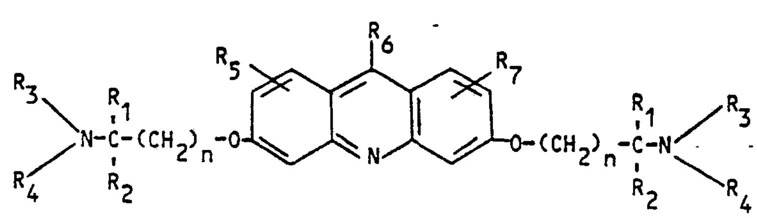

(I)

wherein n is the integer 1, 2 or 3; $R_1$ and $R_2$ are each hydrogen or methyl; $R_3$ and $R_4$ taken together with the

associated N atom are 1-pyrrolidino, 1-piperidino, 1-morpholino or 4-methyl-1-piperazino; and $R_5$, $R_6$ and $R_7$ may be the same or different and are selected from the group consisting of hydrogen, fluoro, chloro, bromo and nitro; the pharmacologically acceptable metal complexes thereof and the pharmacologically acceptable acid-addition salts thereof.

2. A process according to Claim 1 wherein said compound is 3,6-bis[2-(1-piperidinyl)ethoxy]acridine.

3. A process according to Claim 1 wherein said compound is 3,6-bis[3-(1-piperidinyl)propoxy]acridine trihydrochloride.

4. A process according to Claim 1 wherein said compound is 3,6-bis[3-(1-pyrrolidinyl)propoxy]acridine.

5. A process according to Claim 1 wherein said compound is 3,6-bis[3-(4-methyl-1-piperazinyl)-propoxy]acridine.

6. A process according to Claim 1 wherein said compound is 4,5-dibromo-3,6-bis[2-(1-piperidinyl)-ethoxy]acridine.

7. A process according to Claim 1 wherein said compound is 4,5-dichloro-3,6-bis[2-(1-piperidinyl)-ethoxy]acridine.

8. A process according to Claim 1 wherein said compound is 3,6-bis[4-(1-piperidinyl)butoxy]acridine trihydrochloride.

9. A process according to Claim 1 wherein said compound is 3,6-bis[2-(1-pyrrolidinyl)ethoxy]acridine trihydrochloride.

10. A process according to Claim 1 wherein said compound is 4,5-dichloro-3,6-bis[2-(1-pyrrolidinyl)-ethoxy]acridine.

11. A process of preparing the compounds of the formula (I)

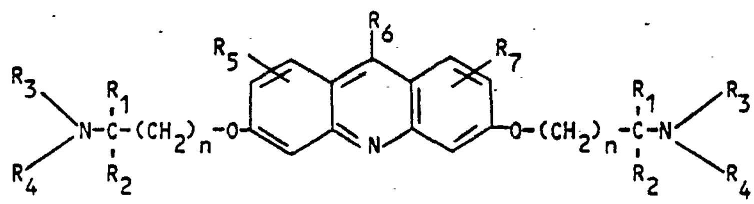

(I)

wherein $R_1$, $R_2$, $R_3$, $R_4$, $R_5$, $R_6$, $R_7$ and n are as defined in claim 1 which comprises condensing a substituted 3,6-acridinediol disodium salt of the formula

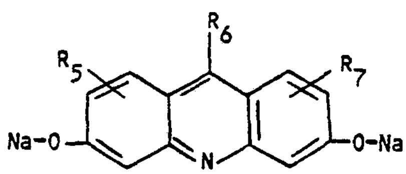

wherein $R_5$, $R_6$ and $R_7$ are as hereinbefore defined with two mole equivalents of an alkyl halide of the formula

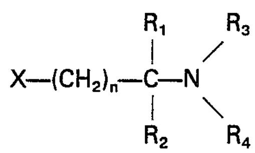

wherein X is chloro or bromo and n, $R_1$, $R_2$ and

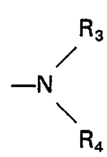

are as hereinbefore defined in an inert solvent at 75°—100°C for a period of time sufficient for a substantial degree of condensation to occur; and, if desired, forming the metal complexes or acid-addition salts thereof.

**Patentansprüche für die Vertragsstaaten: BE CH DE FR GB IT LI NL und SE**

1. Verbindung, ausgewählt aus der Gruppe der Verbindungen der Formel

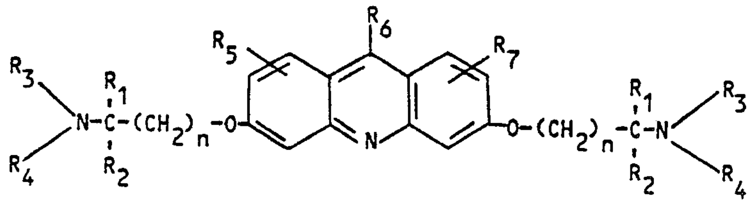

(I)

worin n die Zahl 1, 2 oder 3, $R_1$ und $R_2$ jeweils ein Wasserstoffatom oder eine Methylgruppe sowie $R_3$ und $R_4$ zusammen mit dem daran gebundenen Stickstoffatom eine 1-Pyrrolidino-, 1-Piperidino-, 1-Morpholino- oder 4-Methyl-1-piperazinogruppe bedeuten, und $R_5$, $R_6$ und $R_7$ gleich oder verschieden sind und aus der Gruppe Wasserstoff, Fluor, Chlor- und Bromatom sowie Nitrogruppe ausgewählt sind, und ihre pharmakologisch verträglichen Metallkomplexe und Säureadditionssalze.

2. Verbindungen nach Anspruch 1; 3,6-Bis-[2-(1-piperidinyl)-ethoxy]-acridin und seine pharmakologisch verträglichen Metallkomplexe und Säureadditionssalze.

3. Verbindungen nach Anspruch 1; 3,6-Bis-[3-(1-piperidinyl)-propoxy]-acridin und seine pharmakologisch verträglichen Metallkomplexe und Säureadditionssalze.

4. Verbindungen nach Anspruch 1; 3,6-Bis-[3-(1-pyrrolidinyl)-propoxy]-acridin und seine pharmakologisch verträglichen Metallkomplexe und Säureadditionssalze.

5. Verbindungen nach Anspruch 1; 3,6-Bis-[3-(4-methyl-1-piperazinyl)-propoxy]-acridin und seine pharmakologisch verträglichen Metallkomplexe und Säureadditionssalze.

6. Verbindungen nach Anspruch 1; 4,5-Dibrom-3,6-Bis-[2-(1-piperidinyl)-ethoxy]-acridin und seine pharmakologisch verträglichen Metallkomplexe und Säureadditionssalze.

7. Verbindungen nach Anspruch 1; 4,5-Dichlor-3,6-Bis-[2-(1-piperidinyl)-ethoxy]-acridin und seine pharmakologisch verträglichen Metallkomplexe und Säureadditionssalze.

8. Verbindungen nach Anspruch 1; 3,6-Bis-[4-(1-piperidinyl)-butoxy]-acridin und seine pharmakologisch verträglichen Metallkomplexe und Säureadditionssalze.

9. Verbindungen nach Anspruch 1; 3,6-Bis-[2-(1-pyrrolidinyl)-ethoxy]-acridin und seine pharmakologisch verträglichen Metallkomplexe und Säureadditionssalze.

10. Verbindungen nach Anspruch 1; 4,5-Dichlor-3,6-Bis-[2-(1-pyrrolidinyl)-ethoxy]-acridin und seine pharmakologisch verträglichen Metallkomplexe und Säureadditionssalze.

11. Verfahren zur Herstellung von Verbindungen der Formel (I) gemäß Anspruch 1

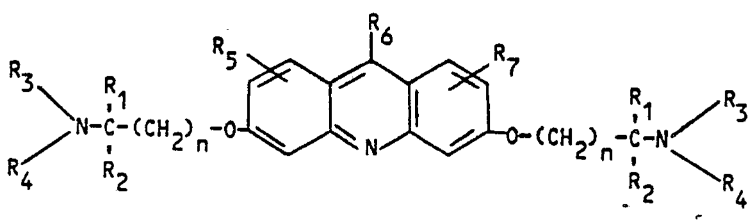

(I)

worin $R_1$, $R_2$, $R_3$, $R_4$, $R_5$, $R_6$, $R_7$ und n wie in Anspruch 1 definiert sind, dadurch gekennzeichnet, daß ein substituiertes 3,6-Acridindioldinatriumsalz der Formel

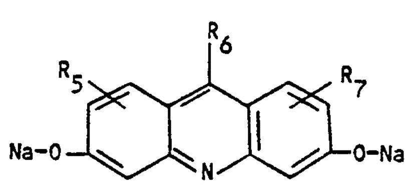

worin $R_5$, $R_6$ und $R_7$ wie vorstehend definiert sind, mit zwei Moläquivalenten eines Alkylhalogenids der Formel

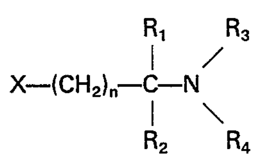

worin X ein Chlor- oder Bromatom und n, $R_1$, $R_2$ und

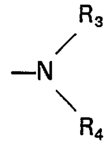

wie vorstehend definiert sind, in einem inerten Lösungsmittel bei 75—100°C während einer Zeit umgesetzt wird, die zum Erreichen eines wesentlichen Kondensationsgrades ausreichend ist, und gewünschtenfalls davon die Metallkomplexe oder Säureadditionssalze gebildet werden.

12. Pharmazeutische Zusammensetzung, enthaltend eine Verbindung nach einem der Ansprüche 1 bis 10 und einen pharmakologisch verträglichen Träger oder ein solches Verdünnungsmittel.

**Patentansprüche für den Vertragsstaat: AT**

1. Verfahren zur Herstellung einer pharmazeutischen Zusammensetzung, die zum Induzieren des Rückgangs von Tumoren bei warmblütigen Tieren und zur Verbesserung des Immunsystems einsetzbar ist, dadurch gekennzeichnet, daß ein pharmakoligisch verträglicher Träger oder ein derartiges Verdünnungsmittel mit Verbindungen der Formel

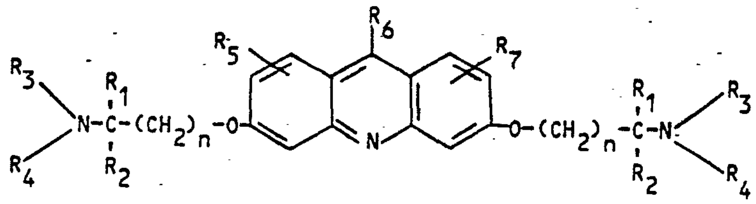

(I)

worin n die Zahl 1, 2 oder 3, $R_1$ und $R_2$ jeweils ein Wasserstoffatom oder eine Methylgruppe, $R_3$ und $R_4$ zusammen mit dem daran gebundenen Stickstoffatom eine 1-Pyrrolidino, 1-Piperidino-, 1-Morpholino- oder 4-Methyl-1-piperazinogruppe bedeuten und $R_5$, $R_6$ und $R_7$ gleich oder verschieden sind und aus der Gruppe bestehend aus Wasserstoff-, Fluor-, Chlor- und Bromatom sowie Nitrogruppe ausgewählt sind, oder mit pharmakologisch verträglichen Metallkomplexen oder Säureadditionssalzen dieser Verbindungen gemischt wird.

2. Verfahren nach Anspruch 1, worin die genannte Verbindung 3,6-Bis-[2-(1-piperidinyl)-ethoxy]-acridin ist.

3. Verfahren nach Anspruch 1, worin die genannte Verbindung 3,6-Bis-[3-(1-piperidinyl)-propoxy]-acridintrihydrochlorid ist.

4. Verfahren nach Anspruch 1, worin die genannte Verbindung 3,6-Bis-[3-(1-pyrrolidinyl)-propoxy]-acridin ist.

5. Verfahren nach Anspruch 1, worin die genannte Verbindung 3,6-Bis-[3-(4-methyl-1-piperazinyl)-propoxy]-acridin ist.

6. Verfahren nach Anspruch 1, worin die genannte Verbindung 4,5-Dibrom-3,6-Bis-[2-(1-piperidinyl)-ethoxy]-acridin ist.

7. Verfahren nach Anspruch 1, worin die genannte Verbindung 4,5-Dichlor-3,6-Bis-[2-(1-piperidinyl)-ethoxy]-acridin ist.

8. Verfahren nach Anspruch 1, worin die genannte Verbindung 3,6-Bis-[4-(1-piperidinyl)-butoxy]-acridintrihydrochlorid ist.

9. Verfahren nach Anspruch 1, worin die genannte Verbindung 3,6-Bis-[2-(1-pyrrolidinyl)-ethoxy]-acridintrihydrochlorid ist.

10. Verfahren nach Anspruch 1, worin die genannte Verbindung 4,5-Dichlor-3,6-Bis-[2-(1-pyrrolidinyl)-ethoxy]-acridin ist.

11. Verfahren zur Herstellung der Verbindung der Formel (I)

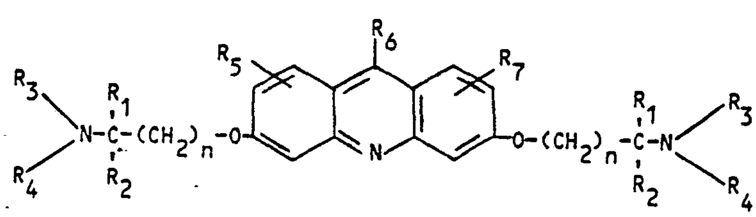

(I)

worin $R_1$, $R_2$, $R_3$, $R_4$, $R_5$, $R_6$, $R_7$ und n gemäß Anspruch 1 definiert sind, dadurch gekennzeichnet, daß ein substituiertes 3,6-Acridindioldinatriumsalz der Formel

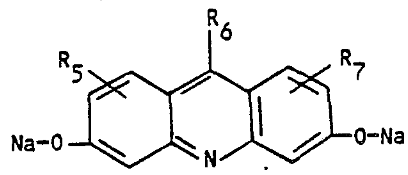

worin $R_5$, $R_6$ und $R_7$ die vorstehende Bedeutung haben, mit zwei Moläquivalenten eines Alkylhalogenids der Formel

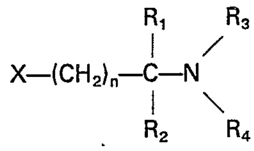

worin X ein Chlor- oder Bromatom sowie n, $R_1$, $R_2$ und

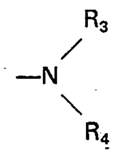

die vorstehende Bedeutung haben in einem inerten Lösungsmittel bei 75—100°C während einer Zeit kondensiert wird, die für das Erreichen eines wesentlichen Kondensationsgrades ausreicht, und gewünschtenfalls die entsprechenden Metallkomplexe oder Säureadditionssalze bildet.

**Revendications pour Etats Contractants: BE CH DE FR GB IT LI NL SE**

1. Un composé choisi dans le groupe constitué par ceux de formule

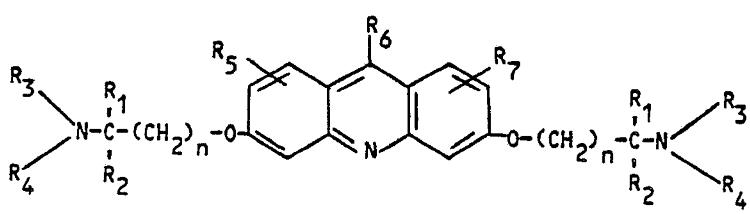

(I)

dans laquelle n est l'entier 1, 2 ou 3; $R_1$ et $R_2$ sont chacun un hydrogène ou un méthyle; $R_3$ et $R_4$ pris ensemble avec l'azote associé sont un 1-pyrrolidino, un 1-pipéridino, un 1-morpholino ou un 4-méthyl-1-pipérazino; et $R_5$, $R_6$ et $R_7$ peuvent être semblables ou différents et sont choisis dans le groupe constitué par un hydrogène, un fluoro, un chloro, un bromo et un nitro; leurs sels métalliques complexes pharmacologiquement acceptables et leurs sels d'addition d'acides pharmacologiquement acceptables.

2. Les composés selon la revendication 1: 3,6-bis[2-(1-pipéridyl)éthoxy]acridine et ses complexes métalliques pharmacologiquement acceptables et ses sels d'addition d'acides pharmacologiquement acceptables.

3. Les composés selon la revendication 1: 3,6-bis[3-(1-pipéridyl)propoxy]acridine et ses complexes métalliques et ses sels d'addition d'acides pharmacologiquement acceptables.

4. Les composés selon la revendication 1: 3,6-bis[3-(1-pyrrolidinyl)propoxy]acridine et ses complexes métalliques et ses sels d'addition d'acides pharmacologiquement acceptables.

5. Les composés selon la revendication 1: 3,6-bis[3-(4-méthyl-1-pipérazinyl)propoxy]acridine et ses complexes métalliques et ses sels d'addition d'acides pharmacologiquement acceptables.

6. Les composés selon la revendication 1: 4,6-dibromo-3,6-bis[2-(1-pipéridyl)éthoxy]acridine et ses complexes métalliques et ses sels d'addition d'acides pharmacologiquement acceptables.

7. Les composés selon la revendication 1: 4,6-dichloro-3,6-bis[2-(1-pipéridyl)éthoxy]acridine et ses complexes métalliques et ses sels d'addition d'acides pharmacologiquement acceptables.

8. Les composés selon la revendication 1: 3,6-bis[4-(1-pipéridyl)butoxy]acridine et ses complexes métalliques et ses sels d'addition d'acides pharmacologiquement acceptables.

9. Les composés selon la revendication 1: 3,6-bis[2-(1-pyrrolidinyl)éthoxy]acridine et ses complexes métalliques et ses sels d'addition d'acides pharmacologiquement acceptables.

10. Les composés selon la revendication 1: 4,5-dichloro-3,6-bis[2-(1-pyrrolidinyl)éthoxy]acridine et ses complexes métalliques et ses sels d'addition d'acides pharmacologiquement acceptables.

11. Le procédé de préparation de composés de formule (I) selon la revendication 1

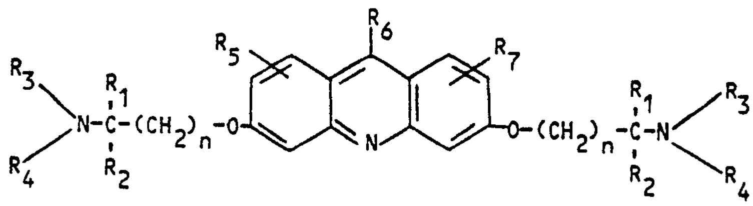

(I)

où $R_1$, $R_2$, $R_3$, $R_4$, $R_5$, $R_6$, $R_7$ et n sont comme défini dans la revendication 1, caractérisé par la réaction d'un sel disodique de 3,6-acridinediol substitué de formule

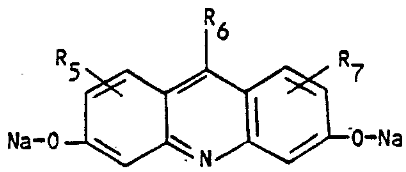

dans laquelle $R_5$, $R_6$ et $R_7$ sont comme précédemment défini avec deux équivalents molaires d'un halogénure d'alkyle de formule

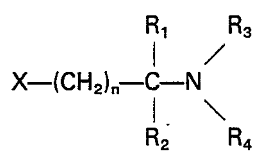

dans laquelle X est un chloro ou un bromo et n, $R_1$, $R_2$ et

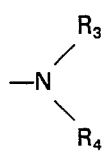

sont comme précédemment défini, dans un solvant inerte entre 75 et 100°C pendand une période suffisante pour qu'un degré notable de condensation se produise; et, si on le désire, la formation des complexes métalliques ou des sels d'addition d'acides de ceux-ci.

12. Une composition pharmaceutique comprenant un composé selon l'une quelconque des revendications 1 à 10 et un support ou diluant pharmacologiquement acceptables.

**Revendications pour l'Etat Contractant: AT**

1. Un procédé pour la préparation d'une composition pharmaceutique utile pour induire la régression des tumeurs chez les animaux à sang chaud et pour renforcer le système immunitaire, caractérisé en ce que l'on mélange un support ou diluant pharmacologiquement acceptables avec les composés de formule

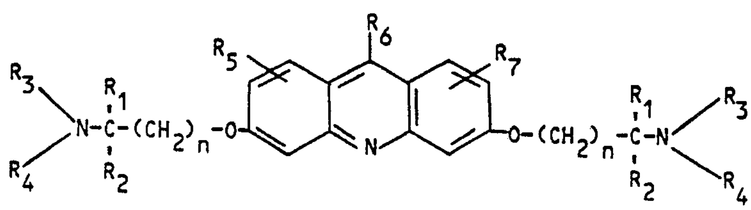

(I)

dans laquelle n est l'entier 1, 2 ou 3; $R_1$ et $R_2$ sont chacun un hydrogène ou un méthyle; $R_3$ et $R_4$ pris ensemble avec l'atome d'azote associé sont un 1-pyrrolidino, un 1-pipéridino, un 1-morpholino ou un 4-méthyl-1-pipérazino; et $R_5$, $R_6$ et $R_7$ peuvent être semblables ou différents et sont choisis dans le groupe constitué par un hydrogène, un fluoro, un chloro, un bromo et un nitro; leurs complexes métalliques pharmacologiquement acceptables et leurs sels d'addition d'acides pharmacologiquement acceptables.

2. Un procédé selon la revendication 1, dans lequel ledit composé est la 3,6-bis[2-(1-pipéridyl)éthoxy-acridine.

3. Un procédé selon la revendication 1, dans lequel ledit composé est le trichlorhydrate de 3,6-bis[3-(1-pipéridyl)propoxy]acridine.

4. Un procédé selon la revendication 1, dans lequel ledit composé est la 3,6-bis[3-(1-pyrrolidinyl)-propoxy]acridine.

5. Un procédé selon la revendication 1, dans lequel ledit composé est la 3,6-bis[3-(4-méthyl-1-pipérazinyl)propoxy]acridine.

6. Un procédé selon la revendication 1, dans lequel ledit composé est la 4,5-dibromo-3,6-bis[2-(1-pipéridyl)éthoxy]acridine.

7. Un procédé selon la revendication 1, dans lequel ledit composé est la 4,5-dichloro-3,6-bis[2-(1-pipéridyl)éthoxy]acridine.

8. Un procédé selon la revendication 1, dans lequel ledit composé est le trichlorhydrate de 3,6-bis[4-(1-pipéridyl)butoxy]acridine.

9. Un procédé selon la revendication 1, dans lequel ledit composé est le trichlorhydrate de 3,6-bis[2-(1-pyrrolidinyl)éthoxy]acridine.

10. Un procédé selon la revendication 1, dans lequel ledit composé est la 4,5-dichloro-3,6-bis[2-(1-pyrrolidinyl)éthoxy]acridine.

11. Un procédé de préparation des composés de formule (I)

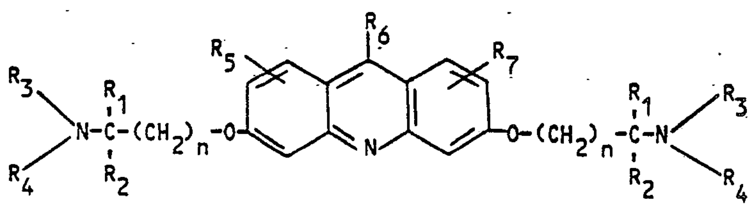

(I)

dans laquelle $R_1$, $R_2$, $R_3$, $R_4$, $R_5$, $R_6$, $R_7$ et n sont comme défini dans la revendication 1, qui comprend la condensation d'un sel disodique de 3,6-acridinediol substitué de formule

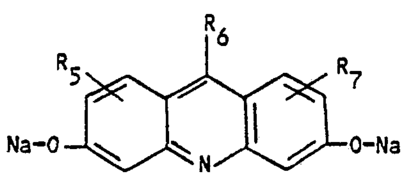

dans laquelle $R_5$, $R_6$ et $R_7$ sont comme précédemment défini, avec deux équivalents molaires d'un halogénure d'alkyle de formule

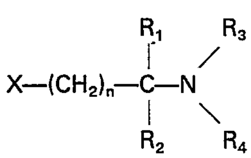

dans laquelle X est un chloro ou un bromo et n, $R_1$, $R_2$ et

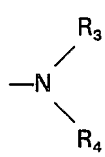

sont comme précédemment défini, dans un solvant inerte entre 75 et 100°C pendant une période suffisante pour qu'un degré notable de condensation se produise; et, si on le désire, la formation de leurs complexes métalliques ou de leurs sels d'addition d'acides.